Europäisches Patentamt

European Patent Office

Office européen des brevets

(11) Publication number: **0 105 669**
**B1**

# EUROPEAN PATENT SPECIFICATION

(45) Date of publication of patent specification: **07.01.87**

(21) Application number: **83305609.6**

(22) Date of filing: **21.09.83**

(51) Int. Cl.⁴: **A 61 F 5/46**

(54) Method of contraception and apparatus for carrying out such method.

(30) Priority: **30.09.82 US 429974**

(43) Date of publication of application:
**18.04.84 Bulletin 84/16**

(45) Publication of the grant of the patent:
**07.01.87 Bulletin 87/02**

(84) Designated Contracting States:
**BE CH DE FR GB IT LI LU NL SE**

(56) References cited:
**EP-A-0 010 812**
**DE-A-2 537 620**
**DE-A-2 913 036**
**GB-A-1 530 565**
**US-A-2 365 296**
**US-A-3 722 500**

(73) Proprietor: **BETA PHASE INC.**
**1060 Marsh Road**
**Menlo Park California 94025 (US)**

(72) Inventor: **Krumme, John F.**
**Woodside**
**San Mateo California (US)**
Inventor: **Hodgson, Darel E.**
**Palo Alto**
**Santa Clara California (US)**
Inventor: **McAdams, Ronald E.**
**Palo Alto**
**Santa Clara California (US)**

(74) Representative: **Hayward, Denis Edward Peter et al**
**Lloyd Wise, Tregear & Co. Norman House**
**105-109 Strand**
**London WC2R 0AE (GB)**

Courier Press, Leamington Spa, England.

## Description

The present invention relates generally to contraceptive techniques and, more particularly, to a method of contraception and apparatus for carrying out such method.

A large number of contraceptive techniques have been developed. Male-utilized condoms and female-utilized diaphragms have been employed for many years but are notably inconvenient and none too effective. Birth control pills are quite effective but have certain adverse side effects that limit their utilization. Intrauterine devices (I.U.D.s) are also generally effective but present problems such as accidental embedding in the uterine wall requiring surgical removal. More recently, devices have been inserted into the oviducts to block or occlude the tube, but, in many cases, require surgical implantation and removal (laparotomy).

In short, all reversible contraceptive techniques, in spite of much development effort, have presented many and varied problems.

It is therefore an object of the present invention to provide a contraceptive method which involves the non-surgical implantation or positioning of a tubal occlusion device in the female oviduct in a manner which assures effective contraception but at the same time preferably enables simple withdrawal of the device when desired.

In the prior art, tubal occlusion devices are known, for obturating ducts in the human body, which consist of hollow resilient-walled plugs that can, when in place, be expanded by the introduction of fluid under pressure from a collapsed generally cylindrical configuration in which they will pass through the duct into a bag or balloon-like configuration in which they seal against the duct walls, such expansion occurring without substantial foreshortening of the plug. More particularly, it has been proposed, for example in GB—A—1530565 and DE—A—2537620, that such plugs should be inserted into the female oviducts, for the purpose of contraception by means of elongated positioning devices that can also serve as a means of introducing the fluid into the plug.

According to the invention, there is provided a tubal occlusion device which comprises a hollow expansible plug composed of resilient material and having an initial elongated generally cylindrical laterally collapsed configuration less than the interior diameter of the tube, and means to effect lateral expansion of said plug into sealing contact with the walls of the tube, characterised in that said plug in its expanded state assumes a foreshortened bellows configuration and can preferably be recollapsed into its elongated generally cylindrical configuration for withdrawal.

Thus, the tubal occlusion device is a hollow expansible plug which can be inserted through the female reproductive tract including the vagina, the uterus, and the narrow and convoluted intramural portion of the oviduct into the isthmic ampullary junction. Since both the intramural and isthmic portions of the oviduct are quite small (e.g. 1—4 mm. in diameter) the occlusion device preferably takes the form of a thin elongated cylinder in its collapsed disposition having a diameter no greater than one millimeter to facilitate passage into the oviduct.

Once properly positioned, the plug is expanded to provide sealing contact with the wall of the oviduct thus effectively preventing conception.

Subsequently, if plug removal is desired, the hollow plug can preferably be collapsed to its thin configuration to allow simple withdrawal.

According to a further aspect, the invention provides contraceptive apparatus which comprises an occlusion device as aforesaid, and an elongate positioning device therefore, with means for releasably connecting the ends of said occlusion device and said positioning device, and actuating means in said positioning device operable when said occlusion device and said positioning device are so connected to actuate the means in the hollow plug of said occlusion device for effecting expansion and/or collapse thereof.

The invention also provides a method of contraception which comprises the steps of non-surgically inserting an occlusion device as aforesaid through the female reproductive tract into the oviduct and expanding the plug of said occlusion device into sealing contact with the wall of the oviduct.

Preferably, the expansible plug is formed of silicone rubber or other resilient material having no adverse interaction in its contact with the oviduct. The expanded bellows-like configuration of the plug not only ensures multiple sealing contact with the walls of the oviduct but also provides frictional engagement effectively precluding longitudinal shifting or dislodgement of the expanded plug in the oviduct.

The plug expansion means is preferably a cooperative arrangement in the plug and the positioning device which can be releasably attached to the plug and is sufficiently elongated so as to pass to the exterior of the female for access thereto.

Preferably also, the positioning device is arranged not only to enable insertion of the occlusion plug but also, when desired, collapse and subsequent withdrawal thereof.

In one embodiment of the invention, the plug is in the form of a hollow bellows composed of silicone rubber closed at its outer or distal end and sealed at its inner proximal end by a connector enabling releasable connection to an elongated positioning device which the physician can manipulate to enable insertion and positioning within the female oviduct. To facilitate such insertion, a length of shape memory metal, such as that known under the trade name Nitinol is positioned in extended generally rectilinear configuration within the hollow plug to deform the same into a lengthened and narrowed configuration. After insertion to the desired position within the oviduct, heat is applied to the shape memory metal which effects contraction thereof longitudinally into a generally helical configuration to cause expansion of the plug laterally into its bellows configuration to effect sealing contact with the walls of the oviduct. Preferably, the heat is generated by passing an electric current through the shape memory metal in an amount sufficient to reach its transition temperature and thus effect contraction of the memory metal. The positioning device can then be released from its connection to the plug, which latter will remain in its laterally expanded configuration in sealing contact with the walls of the oviduct.

A second embodiment of the invention facilitates removal of the rubber plug when desired. This embodiment also includes a hollow bellows-like plug formed of silicone rubber closed at its outer distal end and connected at its inner proximal end to a suitable connector which enables releasable connection to an elongated positioning device. Electrical connections are made from a suitable source to a Nitinol loop within the hollow plug so that when current is applied to heat the Nitinol, it will assume its shape memory position which is an elongated one which essentially narrows the plug dimensions to facilitate insertion into the oviduct. This elongated disposition of the Nitinol acts in opposition to a thin coil spring which tends to contract and thus acts in opposition to the force exerted by the Nitinol elongation. When the applied heat is removed, such coil spring is sufficiently strong to contract and thus force the hollow plug into its bellows-like configuration wherein sealing contact is made with the oviduct. The positioning device can then be released leaving the plug in its contraceptive position within the oviduct.

However, when removal is desired, the positioning device can be again connected to the proximal end of the plug and current applied to heat the Nitinol and thus extend the same, the force of extension of the Nitinol to its memory shape position being substantially greater than that of the coil spring so that once again the Nitinol is extended and the surrounding silicone rubber returned to its collapsed disposition which obviously will facilitate removal of the plug when the contraceptive effect is no longer desired.

In another embodiment of the invention, a mechanical expansion means is employed. More particularly, the occlusive device is again formed of silicone rubber with its outer distal end closed and its inner proximal end sealingly connected to a flexible reservoir also preferably formed of silicone rubber. In its normal position, the plug takes a thin elongated configuration to facilitate insertion into the oviduct. However, the positioning device includes means which encompasses the reservoir and can be actuated to squeeze the same thus to force fluid therefrom into the plug itself and accordingly expand the plug into a bellows configuration to provide sealing contact with the oviduct. The squeezing of the reservoir can then be released but the expanded configuration of the plug itself is retained by a releasable check valve located between the reservoir and the plug itself. The plug forming the occlusion device is thus left in its position until removal of the plug is desired.

When such removal is desired, the positioning device is once again inserted into encompassing relationship with the reservoir and a mechanism at its inner extremity can be actuated to open the releasable check valve so that the pressure within the plug is released and it will return to its narrow cylindrical configuration so that plug removal is facilitated.

Within the hollow plug, there may be positioned a small hollow conduit provided at spaced intervals with lateral openings. Between these openings, annular sealing members are connected between the central conduit and the exterior plug wall, this arrangement causing the plug on expansion to assume a bellows configuration.

Another arrangement for introducing fluid into the plug to expand it includes a hypodermic needle secured at the extremity of the positioning device and which is arranged to penetrate a conventional septum at the proximal end of the plug. After initial insertion and positioning of the plug in a narrow elongated cylindrical form, fluid, hydraulic or pneumatic, can be injected through the needle into the central conduit of the plug so as to expand the silicone rubber plug into a bellows-configuration to seal the oviduct. The positioning device can then be removed and the septum will self-seal and maintain the plug in its expanded contraceptive position.

Subsequently, if the plug is to be removed, the positioning device can be repositioned with the hypodermic needle extending through the septum and with no application of exterior pressure the resiliency of the silicone rubber plug will cause it to expel the fluid therefrom and collapse into its elongated cylindrical configuration to enable the ready removal of the plug by a simple manual pull on the connecting end of the positioning device.

Embodiments of the invention will now be described in more detail by way of example and with reference to the accompanying drawings wherein:

Figure 1 is a diagrammatic sectional view of the female reproductive tract indicating insertion of a tubal occlusion device in accordance with the present invention,

Figure 2 is an enlarged fragmentary perspective view of the occlusion device of Figure 1,

Figure 3 is a perspective view, similar to Figure 2 but showing the occlusion device in its expanded disposition and the positioning device separated therefrom,

Figure 4 is a fragmentary perspective view of a modified occlusion device in its collapsed disposition with its positioning device attached,

Figure 5 is a view similar to Figure 4 with the occlusion device in its expanded disposition,

Figure 6 is a fragmentary perspective view similar to Figures 2 and 4 of a modified embodiment of the invention with the occlusion device in its collapsed disposition,

Figure 7 is a view similar to Figure 6 showing the occlusion unit in its expanded disposition,

Figure 8 is a view similar to Figures 2, 4 and 6 of a further modified embodiment with the occlusion device in its collapsed disposition, and

Figure 9 is a view similar to Figure 8 with the occlusion device in its expanded configuration.

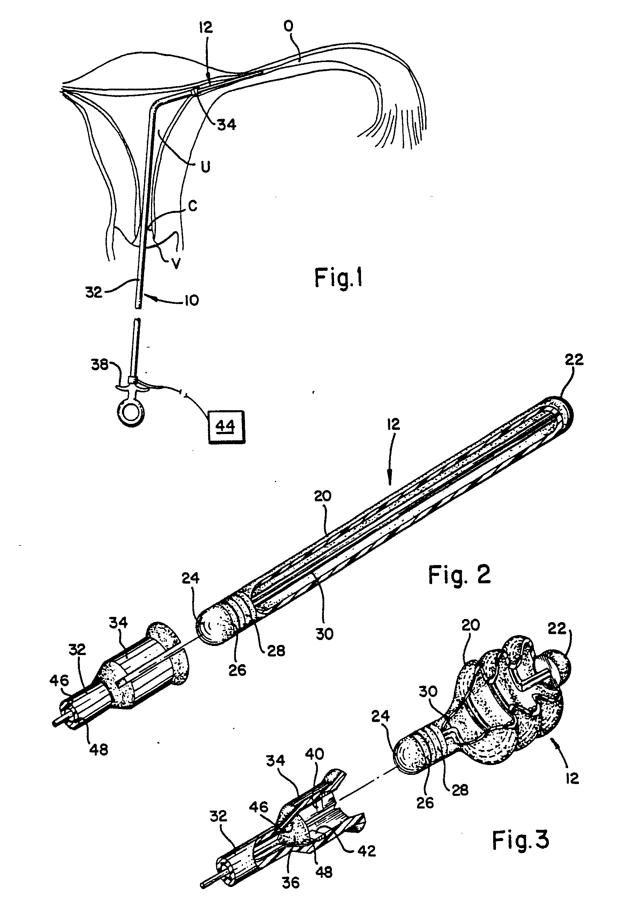

With initial reference to Figure 1 there is illustrated in a sectional showing the reproductive tract of the female including the vaginal channel V, the cervix C, the uterus U and the oviduct or fallopian tubes O.

In accordance with the present invention, an elongated positioning device 10 with the tubal occlusion device 12 detachably mounted at its extremity has been passed upwardly through the female reproductive tract until the occlusion device itself is positioned in the medial portion of the oviduct O. As shown generally in Figure 1 and enlarged perspective in Figure 2, the tubal occlusion device 12 is in the form of a hollow plug 20 of silicone rubber which is shown in its longitudinally-extended and laterally-collapsed disposition wherein its diameter is in the neighborhood of 1 millimeter which facilitates implantation of the plug 20 into the oviduct O which, as previously mentioned, has a diameter in the range of 1 millimeter to 4 millimeters. The outer extremity of the plug 20 is closed by a rounded enclosing tip 22 which also helps to guide the plug into the oviduct when it is inserted by the physician. In turn, the inner end of the hollow plug 20 is sealingly secured to one end of a cylindrical plastic insulated connector 24 also of a very narrow diameter and having two electrodes 26, 28 at spaced positions along its length. These electrodes 26, 28 are joined to the two extremities of a wire loop 30 which is electrically insulated along its length and is formed by thin conductive Nitinol wire which, as shown in Figure 2, is in substantially a lengthened rectilinear disposition which stretches the silicone rubber plug 20 longitudinally so as to laterally effect collapsing of the same into the previously mentioned thin extended disposition with its outer end connected to the tip 22.

The Nitinol wire loop 30 is mechanically deformed to this thin elongated substantially rectilinear configuration at a temperature below its transition temperature in its martensitic phase. However, if the Nitinol is then heated to a temperature above its transition temperature so as to reach its austenitic phase, in accordance with its unique shape memory characteristics, it will automatically deform itself into its preset configuration which in the present instance as best shown in Figure 3 constitutes a foreshortened and widened helical coil which in turn effects foreshortening of the surrounding silicone rubber plug into a bellows-like configuration whose ridges provide periodic contact with the surrounding walls of the oviduct O thus to provide an effective seal and also frictional engagement to preclude movement of the plug along the oviduct channel.

With regard to the Nitinol material itself, details of its characteristics can be found in a NASA publication SP5110 entitled, "55 Nitinol, The Alloy with a Memory; Its Physical Metallurgy, Properties, and Applications" (1972). Briefly, for present purposes, if the Nitinol is a binary compound having between 50.0 and 50.2 percent of Nickel, with the balance being Titanium, the transition temperature would be in the range of 50 to 55 degrees Celsius or 122 to 131 degrees Fahrenheit so that the material can be readily heated to its transition temperature without having any adverse effect on the contacted portions of the reproductive tract. It is to be particularly noted even though the wire is slightly heated above body temperature to provide the transition, it is also enclosed within the silicone rubber so that direct heated contact with the oviduct is not required. It should be additionally mentioned that since 50 degrees Celsius is above normal ambient temperatures, accidental expansion of the plug is avoided.

The positioning device, as shown in Figure 1, is an elongated hollow plastic tube 32 having limited flexibility and a length sufficient so that the plug 20 which is releasably connected to its end can be positioned within the oviduct under control of the physician at a position exterior of the female body. Releasable mechanical connection is made with the connector 24 at the end of the plug 20 by a split-coaxial connector 34 formed by two semi-cylindrical spring fingers also preferably formed by plastic material. The spring action will cause the split fingers to engage the connector 24 at the end of the plug but a conical wedge actuator 36 positioned at the inner end of the coaxial fingers can be pulled by an attached stainless wire puller 38 which extends beyond the end of the positioning tube 32 so that a manual pull by the physician will spread the fingers and allow the connection to the connector end of the plug 20 to be released.

On the interior of the coaxial fingers, electrical contacts 40, 42 are formed at longitudinally-spaced positions so that when the connection with the connector end of the plug is made, the electrodes 26, 28 are engaged so that electrical current supplied from a suitable source indicated at 44 through thin wires 46, 48 within the positioning tube can be energized to effect heating of the Nitinol loop 30 so that it will be heated above its transition temperature and the wire loop will move from its elongated Figure 2 position to the helically-coiled and foreshortened disposition of Figure 3 and, in turn, the surrounding silicone rubber plug 20 will be foreshortened and laterally expanded into contact with the oviduct O as shown clearly in Figure 3. The physician can at this time pull the wire to spread the coaxial fingers of the coaxial connector 34 whereupon the entire positioning device 10 can be withdrawn. The Nitinol loop 30 will retain its coil-like configuration (Figure 3) so that the silicone rubber plug 20 remains in secure sealing contact with the surrounding walls of the oviduct thus precluding conception.

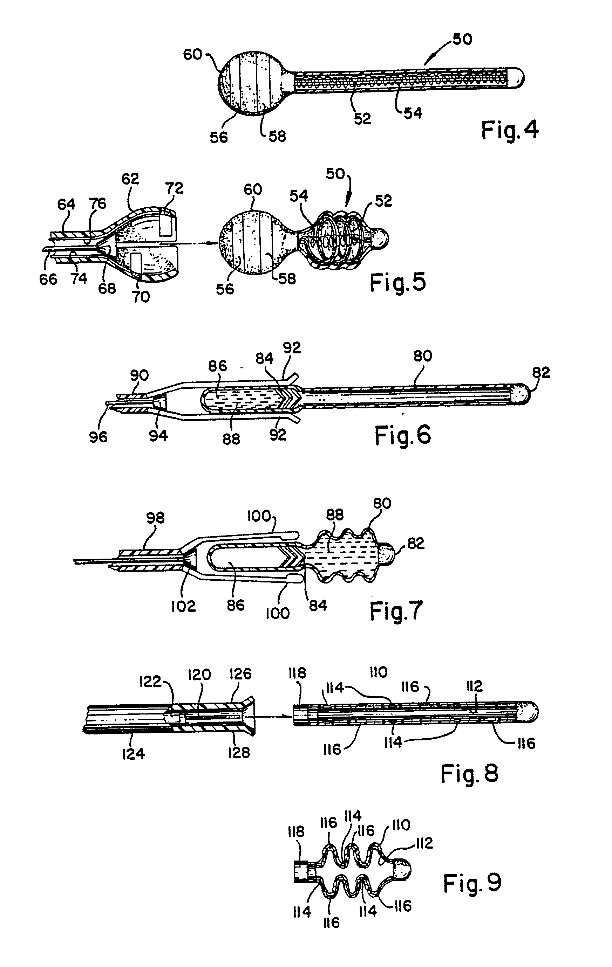

With reference to Figures 4 and 5, a slightly modified embodiment of the invention is illustrated. More particularly, the plug 50 is in the form of a hollow tube of silicone rubber which is movable between an extended thin rectilinear dispositon shown in Figure 4 and a laterally expanded bellows-like configuration wherein sealing contact is made with the walls of the oviduct O in a manner similar to that shown in Figure 1. Within the hollow plug, a loop 52 of Nitinol is again exposed but is arranged so that upon heating above its transition temperature, the loop will attain the longitudinally-extended disposition shown in Figure 4 which will, in turn, cause the surrounding silicone rubber plug to assume a narrowed elongated disposition as shown in the same Figure. Such elongation of the Nitinol loop 52 and the surrounding plug 50 is opposed by a stainless steel coil spring 54 also positioned within the plug whose strength is insufficient to overcome the elongation of the Nitinol loop 52 when heat is applied thereto. However, after heat is removed, it has sufficient strength to overcome the Nitinol elongation and thus force the plug to a foreshortened disposition which, as indicated clearly in Figure 5, expands the plug 50 into its bellows-like configuration wherein sealing contact with the walls of the oviduct are again established.

To provide for heating of the Nitinol, the loop 52 is connected at its extremities to spaced contacts 56, 58 on a ball connector 60 formed of plastic insulating material which is capable of reception within a split spring socket 62 mounted at the end of a positioning tube 64 generally of the same configuration as that described in Figure 1. The spring socket 62 may be opened by pulling on a wire 66 extending through the positioning tube 64 so as to effect a pull on a wedge-like actuator 68 that opens the socket portion enabling release from the ball connector 60 of the plug 50. The interior of the split socket also includes spaced metallic contacts 70, 72 that enable electrical connection to the contacts 56, 58 on the ball when engagement therewith has been achieved through wires 74, 76 extending through the tube to a power source (not shown) under control of the physician as in the first embodiment.

In this embodiment of the invention, the end of the positioning device 64 is releasably connected through the ball and socket arrangement described and current is supplied therethrough to the Nitinol to extend it to its Figure 4 disposition enabling insertion into the oviduct. The electrical source is then disconnected which allows the stainless coil spring 54 to act in a foreshortening manner within the plug 50 so that it attains the bellows-like configuration shown in Figure 5 whereupon the positioning device can be removed leaving the plug in its contraceptive disposition.

However, it should be noted that if the plug 50 is to be removed for any reason, the positioning device 64 can again be connected to the end thereof and current applied which will supply sufficient heating of the Nitinol to again extend it to its Figure 4 position so that ready removal of the plug 50 from the oviduct is enabled.

While the described embodiments of the invention both utilized Nitinol and electrical heating thereof, it will be apparent to those skilled in the art that other forms of heating can be used, direct conductive heating of the Nitinol, inductive heating or many other alternatives.

Furthermore, other means can be provided to expand or collapse the tubal occlusion device without the use of Nitinol and, more particularly, with a simple mechanical activation. By way of example, reference is made to Figures 6 and 7 where a simple hydraulic arrangement is utilized. As shown in Figure 6, the device includes again a hollow cylindrical plug 80 in its collapsed disposition having a relatively thin elongated disposition with a closed rounded tip 82, the same being formed by silicone rubber of similar material having a predetermined resiliency and the characteristic of not interacting adversely with the walls of the oviduct in which it is again implanted in the manner generally shown in Figure 1. The inner end of the silicone rubber plug 80 is connected through a releasable one-way valve 84 to a closed hydraulic reservoir 86 also composed of flexible material which normally is just in one-way fluid contact with the plug itself. The reservoir 86 is filled with some hydraulic fluid 88 and if pressure is applied to such reservoir the fluid will be delivered through the one-way valve 84 into the plug 80 so as to exert interior pressure which expands the plug into its laterally-expanded, bellows-like configuration as shown in Figure 7. The bellows-like configuration is achieved by employing a plug having the internal arrangements described hereinafter with reference to Figures 8 and 9.

To exert this pressure, an elongated positioning tube 90 includes resilient squeezer tips 92 at its inner end that can be moved into encompassing relationship with the reservoir 86 and thence through the release of a wedge actuator 94 connected to a wire 96 encompassed within the positioning plastic tube be released so that the spring squeezer tips 92 will move inwardly into the position shown in Figure 6 so as to eject the fluid 88 through the one-way valve 84 into the occlusion plug 80 to expand the same into its bellows-like configuration where sealing of the oviduct passage is accomplished.

In turn, if the plug 80 is to be collapsed to enable removal, another positioning tube 98 as shown in Figure 7 can be positioned over the reservoir with the spring fingers 100 encompassing the one-way valve 84. When a wedge actuator 102 is released, the one-way valve is opened whereupon the inherent resiliency of the silicone rubber tube 80 will exhaust the fluid back into the flexible reservoir 86 thus to reduce the lateral dimensions of the plug into the Figure 6 disposition whereby ready withdrawal can be achieved.

Yet another mechanical fluid unit is illustrated in Figures 8 and 9 wherein again a hollow plug 110 is formed by silicone rubber of a hollow configuration which normally will reside in a narrow elongated disposition such as shown in Figure 8 with the outer end of the silicone rubber plug closed and a central conduit 112 positioned therewithin that is sealed to the surrounding silicone rubber at longitudinal intervals by annular partitions 114. Between such partitions, the conduit 112 has lateral openings 116 which enable fluid connection between the annular space between the partitions and a source of hydraulic fluid. The inner or proximal end of the plug 110 is closed by a septum 118 which can be penetrated by a hypodermic needle 120 secured to the end of a conduit 122 within a positioning tube 124 of the type generally described in connection with the previous embodiments of the invention so that fluid, liquid or gaseous can be injected from the exterior of the female through the needle 120 into the central conduit 112 so as to expand the silicone rubber to the bellows-like configuration shown in Fig. 9. Split encompassing spring members 126, 128 connected to the end of the positioning tube 124 surround the hypodermic needle and allow connection to the portion of the plug 110 surrounding the septum to enable the initial positioning and also to subsequently enable positioning of the needle through the septum when removal of the plug is desired. In such case, the inherent resiliency of the silicone rubber will force the fluid therewithin out of the plug 110 and if necessary, a vacuum connection (not shown) can be made to the exterior end of the hypodermic needle to accelerate withdrawal of fluid from the plug and its collapse to the disposition shown in Figure 8 whereby easy withdrawal of the occlusion plug can be achieved.

### Claims

1. A tubal occlusion device which comprises a hollow expansible plug composed of resilient material and having an initial elongated generally cylindrical laterally collapsed configuration less than the interior diameter of the tube, and means to effect lateral expansion of said plug into sealing contact with the walls of the tube, characterised in that said plug in its expanded state assumes a foreshortened bellows configuration and can preferably be recollapsed into its elongated generally cylindrical configuration for withdrawal.

2. A tubal occlusion device according to Claim 1 wherein said plug is composed of silicone rubber.

3. A tubal occlusion device according to Claim 1 or Claim 2, further comprising means for introducing fluid under pressure into said hollow resilient plug to effect expansion thereof.

4. A tubal occlusion device according to Claim 3, which comprises a central conduit extending through said hollow resilient plug and having longitudinally spaced, lateral openings in its wall, and

a plurality of annular partitions connecting said central conduit intermediate its openings to the interior wall of said hollow plug,

said central conduit having at one end means for connection to a source of fluid under pressure.

5. A tubal occlusion device according to Claim 4, which comprises valve means adjacent said end of said central conduit for controlling communication with the fluid source.

6. A tubal occlusion device according to Claim 3 or Claim 4 or Claim 5, further comprising an enclosed resilient reservoir connected to one end of said plug and containing a fluid which is expellable from the reservoir into the plug by the application to the reservoir of exterior compressive force.

7. A tubal occlusion device according to Claim 1 or claim 2, which comprises a member of shape-memory material extending longitudinally within said hollow plug to initially hold said plug in its laterally-collapsed condition but which changes its configuration upon the application of heat thereto to effect lateral expansion of said plug into its foreshortened bellows configuration.

8. A tubal occlusion device according to Claim 1 or Claim 2, which comprises a member inside the hollow plug composed of shape-memory material that when heated takes up a configuration such that it extends longitudinally within the plug to hold the plug in the laterally-collapsed condition; and spring means automatically operative upon removal of the heat to effect lateral expansion of said plug into its foreshortened bellows configuration.

9. A tubal occlusion device according to Claim 7 or Claim 8 wherein said shape-memory material is a nickel/titanium alloy.

10. A tubal occlusion device according to Claim 7 or Claim 8 or Claim 9, wherein said shape-memory material is electrically conductive, and means is provided for connecting the same to an external source of electrical power to effect heating thereof.

11. A tubal occlusion device according to Claim 10, wherein said hollow plug is composed of electrically non-conductive material.

12. Contraceptive apparatus which comprises a tubal occlusion device according to any one of Claims 1 to 11, and an elongated positioning device therefore, with means for releasably connecting the ends of said occlusion device and said positioning device, and actuating means in said positioning device operable when said occlusion device and said positioning device are so connected to actuate the means in the hollow plug of said occlusion device for effecting expansion and/or collapse thereof.

13. Contraceptive apparatus according to Claim 12, wherein said connecting means includes spring means for establishing the releasable connection and manually-operable means for releasing the connection.

14. Contraceptive apparatus according to Claims 10 and 12, or Claims 10 and 13, wherein said actuating means includes means for supplying electrical current from a source of electrical power to said electrically-conductive shape-memory material member.

15. Contraceptive apparatus according to Claims 3 and 12, or Claims 3 and 13, wherein said actuating means includes means for introducing fluid into the hollow plug of said occlusion device.

16. A method of contraception which comprises the steps of non-surgically inserting an occlusion device according to any one of Claims 1 to 11 through the female reproductive tract into the oviduct, and expanding the plug of said occlusion device into sealing contact with the wall of the oviduct.

17. A method of contraception according to Claim 16, which comprises the additional step of collapsing the plug for non-surgical withdrawal.

18. A method of contraception according to Claim 16 or Claim 17, wherein the occlusion device is releasably connected to an elongate positioning device for insertion or removal.

**Patentansprüche**

1. Tubenokklusionsvorrichtung bestehend aus einem ausdehnbaren Stopfen, der aus elastischem Werkstoff besteht und eine eingangs längliche, allgemein zylinderförmig seitlich zusammenfaltbare Gestalt aufweist, die kliener als der Tubeninnendurchmesser ist, und aus einer Einrichtung, durch die eine seitliche Ausdehnung des Stopfens bewirkt wird, so daß sich ein dichtender Kontakt mit den Tubenwänden ergibt, dadurch gekennzeichnet, daß der Stopfen in ausgedehntem Zustand eine verkürzte faltenbalgartige Gestalt annimmt und vorzugsweise zur Herausnahme wieder zusammenfaltbar ist in seine längliche, allgemein zylinderförmige Gestalt.

2. Tubenokklusionsvorrichtung nach Anspruch 1, dadurch gekennzeichnet, daß der Stopfen aus Silikongummi gefertigt ist.

3. Tubenokklusionsvorrichtung nach Anspruch 1 oder 2, gekennzeichnet durch Mittel zum Einführen eines unter Druck stehenden Strömungsmittels in den hohlen elastischen Stopfen, der dadurch ausgedehnt wird.

4. Tubenokklusionsvorrichtung nach Anspruch 3, gekennzeichnet durch einen Zentralkanal, der sich durch den hohlen elastischen Stopfen erstreckt und an seinen Wänden in Längsrichtung in Abstand angeordnete seitliche Öffnungen aufweist, und durch eine Anzahl von ringförmigen Trennwänden, durch die der Zentralkanal zwischen dessen Öffnungen mit der Innenwand des hohlen Stopfens verbunden wird, wobei der Zentralkanal an einem Ende Mittel zum Anschließen an eine Druckmittelquelle aufweist.

5. Tubenokklusionsvorrichtung nach Anspruch 4, dadurch gekennzeichnet, daß zum Steuern das Anschlußes an die Strömungsmittelquelle Ventilmittel neben dem Ende des Zentralkanals vorgesehen sind.

6. Tubenokklusionsvorrichtung nach einem der vorhergehenden Ansprüche 3, 4 oder 5, gekennzeichnet durch einen an einem Ende des Stopfen liegenden geschlossenen elastischen Vorratsbehälter mit einem Strömungsmittel, das durch Anlegen eines Aussendrucks an den Vorratsbehälter aus diesem Behälter in den Stopfen ausgetrieben werden kann.

7. Tubenokklusionsvorrichtung nach einem der vorhergehenden Ansprüche 1 oder 2, gekennzeichnet durch ein Teil aus einem Werkstoff mit Gedächniseffekt, das sich in Längsrichtung innerhalb des hohlen Stopfen erstreckt, wodurch der Stopfen eingangs in dessen seitlich zusammengefalteten Zustand gehalten wird, wobei jedoch dessen Gestalt nach Erwärmen desselben dahingehend verändert wird, daß die seitliche Ausdehnung des Stopfens zu der verkürzten faltenbalgartigen Gestalt führt.

8. Tubenokklusionsvorrichtung nach Anspruch 1 oder 2, gekennzeichnet durch ein Teil im Innern des hohlen Stopfens aus einem Werkstoff mit Gedächniseffekt, das bei Erwärmung eine Gestalt annimmt, so daß es längs innerhalb des Stopfens verläuft und dabei den Stopfen in dessen seitlich zusammengefalteten Zustand hält, und durch federnde Mittel, die selbsttätig nach der Wärmewegnahme wirksam werden, so daß bei seitlicher Ausdehnung des Stopfens dieser die verkürzte faltenbalgartige Gestalt annimmt.

9. Tubenokklusionsvorrichtung nach einem der vorhergehenden Ansprüche 7 oder 8, dadurch gekennzeichnet, daß als Werkstoff mit Gedächniseffekt eine Legierung aus Nickel/Titanium verwendet wird.

10. Tubenokklusionsvorrichtung nach einem der vorhergehenden Ansprüche 7, 8 oder 9, dadurch gekennzeichnet, daß der Werkstoff mit Gedächniseffekt elektrisch leitend ist und daß Mittel vorgesehen sind, durch die dieser an eine externe elektrische Stromquelle zum Zwecke der Aufheizung anschließbar ist.

11. Tubenokklusionsvorrichtung nach Anspruch 10, dadurch gekennzeichnet, daß der hohle Stopfen aus elektrisch nicht leitendem Werkstof gefertigt ist.

12. Mechanismus zur Empfängnisverhütung bestehend aus der Tubenokklusionsvorrichtung nach einem der vorhergehenden Ansprüche 1 bis 11, gekennzeichnet durch eine dafür verwendbare längliche Positionierungseinrichtung mit Mitteln, durch die die Enden der Okklusionsvorrichtung und der Positionierungseinrichtung lösbar angeschlossen werden können, und durch Stellglieder in der Positionierungseinrichtung, die wirksam werden, wenn die Okklusionsvorrichtung und die Positionierungseinrichtung so angeschlossen sind, daß die Mittel im hohlen Stopfen der Okklusionsvorrichtung wirksam werden und dessen Ausdehnung und/oder Zusammenfalten bewirken.

13. Mechanismus zur Empfängnisverhütung nach Anspruch 12, dadurch gekennzeichnet, daß als Anschlußmittel Federn, durch die der lösbare Anschluß hergestellt wird, und von Hand zu betätigende Mittel verwendet werden, durch die der Anschluß gelöst wird.

14. Mechanismus zur Empfängsnisverhütung nach den vorhergehenden Ansprüchen 10 und 12 oder 10 und 13, dadurch gekennzeichnet, daß als Stellglieder Mittel zum Einspeisen eines elektrischen Stroms aus einer Stromquelle an das elektrisch leitende Teil aus Werkstoff mit Gedächniseffekt vorgesehen sind.

15. Mechanismus zur Empfängnisverhütung nach den vorhergehenden Ansprüchen 3 und 12 oder 3 und 13, dadurch gekennzeichnet, daß als Stellglieder Mittel zum Einführen des Strömungsmittel in den hohlen Stopfen der Okklusionsvorrichtung vorgesehen sind.

16. Verfahren zur Empfängnisverhütung gekennzeichnet durch nicht chirurgisches Einführen einer Okklusionsvorrichtung nach einem der vorhergehenden Ansprüche 1 bis 11 durch den Genitaltrakt zum Eileiter, wobei der Stopfen der Okklusionsvorrichtung derart ausgedehnt wird, daß er sich an die Eileiterwand dichtend anlegt.

17. Verfahren zur Empfängnisverhütung nach Anspruch 16, dadurch gekennzeichnet, daß man den Stopfen sich zusammenfalten läßt, so daß er auf nicht chirurgische Weise entfernt werden kann.

18. Verfahren zur Empfängnisverhütung nach einem der vorhergehenden Ansprüche 16 oder 17, dadurch gekennzeichnet, daß die Okklusionsvorrichtung zum Einführen und Entfernen lösbar mit einer länglichen Positionierungseinrichtung verbunden ist.

**Revendications**

1. L'appareil de blocage du tube qui comprent une soupape dilatable soufflet composée de matériel élastique et ayant un allongement initiale générale une configuration cylindrique latéralement affaissee inférieure du diamêtre interme du tube, et les moyens d'effecteur une dilatation latérale de la dite soupape dans le contacte scellé avec les parrois du tube, caractérisé dans le dite soupape dans son état de dilatation prend une configuration soufflet raccourci et peut de preferance être re-affaissée dans son allongement généralement dans une configuration cylindrique pour son retrait.

2. L'appareil de blocage du tube suivant la revendication 1 dans laquelle la dite soupape est composée de caoutchouc de silicone.

3. L'appareil de blocage du tube suivant la revendication 1 ou 2 comprend en plus les moyens pour l'introduction du fluide sous pression dans la dite soupape élastique en soufflet pour effectuer le dilatement de celui-ci.

4. L'appareil de blocage du tube suivant la revendication 3, qui comprend un conduit central s'étendant par la dite soupape élastique soufflet et ayant un espace dans la longueur des ouvertures latérales dans ses parrois et une pluralité de partitions en anneau connectée dans le dit conduit central intermédiaire ses ouvertures étant à l'intérieur des parrois de la dite soupape soufflet, le dit conduit central ayant à une de ses extrémités des moyens de connection à la source du fluide sous-pression.

5. L'appareil de blocage du tube qui suivant la revendication 4, inclue les moyens de valve adjacente de la dite extrémité du dit conduit central pour le controle de communication avec la source du fluide.

6. L'appareil de blocage du tube suivant la revendication 3 ou 4 ou 5 comprend en plus un réservoir élastique fermé, ayant une de ses extrémités en relation avec la dite soupape et contenant un fluide qui est expulsable du réservoir dans la soupape par l'application au réservoir de force compressive externe.

7. L'appareil de blocage du tube suivant la revendication 1 ou 2 qui comprend un membre du matériel en forme de memoire extensible dans le sens de la longueur à l'intérieur; de la dite soupape soufflet qui tient initialement la dite soupape dans sa condition affaissée latéralement mais qui change de configuration lors de l'application de chaleur et donc affecte la dilatation latérale de la dite soupape dans sa configuration soufflet raccourcie.

8. L'appareil de blocage du tube suivant la revendication 1 ou 2 qui comprend un membre à l'intérieur de la soupape soufflet composé de matériel en forme de mémoire qui lorsque l'on le chauffe prend une configuration telle qui s'allonge dans la soupape pour tenir la soupape dans sa condition affaissée latéralément, et les moyens de ressort automatique se mettent en action lorsque l'on enleve la source de chaleur pour l'effet de dilatation latérale de la dite soupape dans sa configuration soufflet raccourcie.

9. L'appareil de blocage du tube suivant la revendication 7 ou 8 dans laquelle le dit matériel en forme de memoire est en nickel/mélange de titane.

10. L'appareil de blocage du tube suivant la revendication 7 ou 8 ou 9 ou dans le dit matériel en forme de mémoire est électriquement conducteur, et les moyens sont fournis pour connecter celui-ci a une source externe de puissance electrique pour effectuer le chauffage.

11. L'appareil de blocage du tube suivant la revendication 10 dans laquelle la dite soupape soufflet est composée de matériel électriquement non-conducteur.

12. L'appareil contraceptif qui comprend un appareil de blocage du tube suivant l'une ou autre des revendications 1 a 11, qui donc posséde un appareil de positionement allongé, avec les moyens de relacher les extrémités de connection du dit appareil de blocage et du dit appareil de positionement, et, les moyens de mise en action dans le dit appareil de positionement operable lorsque le dit appareil de blocage et le dit appareil de positionement sont en fait connecté pour actionner les moyens dans la soupape soufflet du dit appareil de blocage pour effectuer la dilatation ou l'affaissage suivant le cas.

13. L'appareil contraceptif suivant la revendication 12, dans laquelle les moyens de connection comprennent les moyens de ressorts pour établir le relachage de la connection et possédant les moyens pour être opérer manuellement pour le relachage de la connection.

14. L'appareil contraceptif suivant les revendications 10 et 12 ou les revendications 10 et 13, dans lesquelles les moyens de mise en action comprennent des moyens d'approvisionnement en courant electrique au dit menbre de matériel en forme de mémoire électriquement conducteur.

15. L'appareil contraceptif suivant les revendications 3 et 12, ou revendications 3 et 13, dans lesquelles les dits moyens de mise en action comprennent les moyens pour l'introduction de fluide dans la soupape soufflet du dit appareil de blocage.

16. Une méthode de contraception qui comprend aucune etape d'insertion chirugicale d'un appareil de blocage suivant une ou plusieurs des reclamations 1 a 11 à travers la voie reproductive femelle dans l'oviducte, et la dilatation de la soupape du dit appareil de blocage lors du contact de scellage avec la parroi de l'oviducte.

17. Une méthode de contraception suivant la revendication 16 qui comprend l'étape additionelle de l'affaissement de la soupape pour un retrait non-chirugical.

18. Une méthode de contraception suivant la revendication 16 ou 17 dans lesquelles l'appareil de blocage est les connections sont relachables pour le positionement allongé de l'appareil pour l'insertion ou le retrait.

Fig.1

Fig. 2

Fig.3

0 105 669

Fig. 4

Fig.5

Fig.6

Fig.7

Fig. 8

Fig.9

2